(19) Europäisches Patentamt
European Patent Office
Office européen des brevets

(11) Veröffentlichungsnummer: **0 173 976**
**B1**

(12) # EUROPÄISCHE PATENTSCHRIFT

(45) Veröffentlichungstag der Patentschrift: **22.07.87**

(51) Int. Cl.⁴: **C 07 C 69/675,** C 07 C 67/58

(21) Anmeldenummer: **85110925.6**

(22) Anmeldetag: **30.08.85**

(54) **Verfahren zur Gewinnung von Hydroxypivalinsäureneopentylglykolester.**

(30) Priorität: **05.09.84 DE 3432577**

(43) Veröffentlichungstag der Anmeldung: **12.03.86 Patentblatt 86/11**

(45) Bekanntmachung des Hinweises auf die Patenterteilung: **22.07.87 Patentblatt 87/30**

(84) Benannte Vertragsstaaten: **DE FR GB IT NL**

(56) Entgegenhaltungen:
**DE-A-2 234 358**
**DE-A-2 500 311**

(73) Patentinhaber: **BASF Aktiengesellschaft, Carl-Bosch- Strasse 38, D-6700 Ludwigshafen (DE)**

(72) Erfinder: **Merger, Franz, Dr., Max- Slevogt- Strasse 25, D-6710 Frankenthal (DE)**
Erfinder: **Hettinger, Peter, Dr., Schloss- Strasse 3, D-6803 Edingen- Neckarhausen (DE)**
Erfinder: **Weber, Theodor, Virchowstrasse 20, D-6700 Ludwigshafen (DE)**
Erfinder: **Boettger, Guenter, Dr., Langen Wingert 16 b, D-6702 Bad Duerkheim (DE)**
Erfinder: **Koernig, Wolfgang, Dr., Dachsweg 5, D-6901 Dossenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 173 976 B1

LIBER, STOCKHOLM 1987

**Beschreibung**

Die vorliegende Erfindung betrifft ein verbessertes Verfahren zur Gewinnung von Hydroxypivalinsäureneopentylglykolester (I) aus Reaktionsgemischen, die man bei der Umsetzung von Hydroxypivalinaldehyd (II) in Gegenwart von Calcium-, Barium- oder Strontiumhydroxid als Katalysator und anschließende Säurebehandlung erhält, durch Abtrennung der hierbei entstehenden Salze und destillative Aufarbeitung des verbleibenden Gemisches auf (I).

Die Darstellung von (I) durch Umsetzung (Disproportionierung) nach Tischtschenko in Gegenwart katalytischer Mengen von Calcium-, Barium-oder Strontiumhydroxid gemäß folgender Gleichung

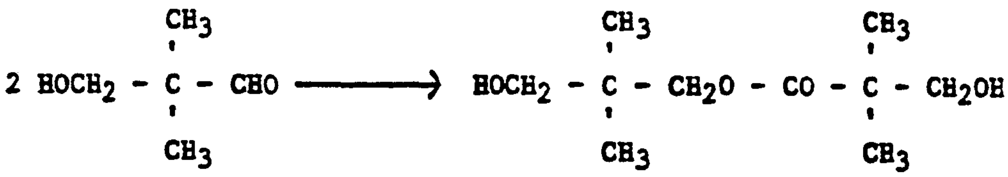

(II) (I)

ist allgemein bekannt. Weiterhin ist es bekannt, z.B. aus der DE-OS 22 34 358, die hierbei erhältlichen Reaktionsgemische vor ihrer Aufarbeitung auf (I) zweckmäßigerweise anzusäuern, wobei das entsprechende Salz erhalten wird. Die Gewinnung von (I) aus dem salzhaltigen Reaktionsgemisch bereitet jedoch Schwierigkeiten, da die Salze teils in gelöster, teils in ungelöster Form vorliegen. Soweit sie fest sind, müssen sie abfiltriert werden, eine Maßnahme, die verfahrenstechnisch stets problematisch ist, und soweit sie gelöst bleiben, fallen sie bei der Destillation aus und bilden Verkrustungen in den Destillationsapparaturen, die wiederum, insbesondere wenn in großtechnischem Maßstab gearbeitet wird, schlechte Wärmeübertragung und Zersetzung des Wertproduktes herbeiführen.

Nach dem Verfahren der DE-OS 22 34 358 wurde das Problem der Salzabtrennung in der Weise gelöst, daß man die Base mittels Ameisensäure in das Formiat überführte, des sodann abfiltriert werden konnte, da es im Reaktionsgemisch weitgehend unlöslich ist.

Da die durch die Filtration bedingten Nachteile damit aber nicht behoben wurden, lag der Erfindung die Aufgabe zugrunde, die Gewinnung von (I) aus dem Reaktionsgemisch nach Tischtschenko verfahrenstechnisch einfacher und damit wirtschaftlicher zu gestalten.

Demgemäß wurde ein verbessertes Verfahren zur Gewinnung von Hydroxypivalinsäureneopentylglykolester (2,2-Dimethyl-1,3-propandiol-hydroxypivalinsäure-monoester) aus Reaktionsgemischen, die man bei der Umsetzung von Hydroxypivalinaldehyd in Gegenwart von Calcium-, Barium- oder Strontiumhydroxid als Katalysator und anschließende Säurebehandlung erhält, durch Abtrennung der hierbei entstehenden Salze und destillative Aufarbeitung des verbleibenden Gemisches auf Hydroxypivalinsäureneopentylglykolester gefunden, welches dadurch gekennzeichnet ist, daß man im Reaktionsgemisch zur Entfernung der Salze einen Wassergehalt von mindestens 20 % und höchstens 60 % einstellt und die Salze bei Temperaturen von 50 bis 100°C extrahiert.

Das gute Gelingen dieses Verfahrens ist bemerkenswert, weil es im Hinblick auf die Wasserlöslichkeit der Reaktionspartner (die Löslichkeit des Wertproduktes (I) in Wasser nimmt mit steigender Temperatur zu) nicht zu erwarten war, daß eine befriedigende Trennung in eine salzhaltige wäßrige und eine weitgehend salzfreie organische Phase stattfindet.

Für die Säurebehandlung eignen sich Säuren, die gut wasserlöslich sind und vorzugsweise solche, die stärker sind als Hydroxypivalinsäure, also z.B. Salzsäure, Essigsäure, Zitronensäure und vor allem Ameisensäure.

Dem nach Tischtschenko erhaltenen Reaktionsgemisch, das schwach basisch reagiert, wird zur Vervollständigung der Neutralisation vor der wäßrigen Extraktion 0,4 bis 3,0 mol, insbesondere 0,5 bis 2,0 mol Säure, bezogen 1 mol Katalysator zugegeben, so daß das Gemisch schwach sauer oder vorzugsweise neutral reagiert. Die Säure kann unverdünnt oder in Form ihrer wäßrigen Lösung verwendet werden, die Säurezugabe erfolgt bei einer Temperatur von mindestens 50, vorteilhaft 60 bis 80°C.

Zur Extraktion wird dem Reaktionsgemisch bei Temperaturen von 50 bis 100, vorteilhaft 60 bis 80, insbesondere 65 bis 75°C soviel Wasser zugesetzt, wie zum Lösen der Salze erforderlich ist. In der Regel wird dies durch Einstellen eines Wassergehalts von 20 bis 60 %, vorteilhaft 20 bis 40 % erreicht.

Die erfindungsgemäße Maßnahme, die Extraktion bei erhöhter Temperatur durchzuführen, beruht nicht allein auf einen Anstieg der Salzlöslichkeit mit der Temperatur, sondern insbesondere darauf, daß nur bei Temperaturen über 50°C eine Phasentrennung in eine salzhaltige wäßrige und eine weitgehend salzfreie organische Phase innerhalb einer vernünftigen Zeit, in der Regel innerhalb von 10 bis 30 min, möglich ist.

Bei Verwendung von Essigsäure zur Neutralisation des Reaktionsgemisches würde es sich z.B. anbieten, bei möglichst niedriger Temperatur zu extrahieren, da die Acetate ein inverses Lösungsverhalten zeigen, das hätte aber zur Folge, daß selbst nach Stunden noch eine milchige Emulsion vorliegt. Erst die Erhöhung der

Temperatur führt zu einer Phasentrennung.

Die Durchführung der Extraktion erfolgt nach den dafür üblichen Techniken kontinuierlich oder diskontinuierlich, so daß sich weitere Angaben hierüber erübrigen.

Nach dem erfindungsgemäßen Verfahren liegt der Entsalzungsgrad der organischen Phase bei 80 bis 90 %, bezogen auf die eingesetzte Menge Hydroxid. Eine weitere Abreicherung auf 5 % der Salze im rohen Endstoff läßt sich durch wiederholte Extraktion unter gleichen Bedingungen erzielen. Die in der Zweitextraktion anfallende wäßrige Phase wird zur Vermeidung von Ausbeuteverlusten von (I) zweckmäßig in die erste Extraktionsstufe eines weiteren Ansatzes eingebracht.

Das nach der Extraktion anfallende Rohprodukt läßt sich durch fraktionierende Destillation nach den dafür üblichen Techniken isolieren. Aufgrund des geringen Salzgehaltes des Rohproduktes kann man auch vorteilhaft einen Dünnfilmverdampfer verwenden, wodurch das Wertprodukt (I) in hoher Reinheit und hoher isolierter Ausbeute anfällt.

(I) dient bekanntermaßen als wertvoller Ausgangsstoff für die Herstellung von Polyestern, Kunstharzen und Weichmachern.

**Beispiel 1**

101 Teile 37 %ige wäßrige Formaldehydlösung und 99 Teile Isobutyraldehyd wurden bei 40°C unter Stickstoff durch Rühren mit 4,2 Teilen 40 %iger wäßriger Trimethylaminlösung gemischt. Die Temperatur des Gemisches stieg unter Rückflußkühlung im Verlauf von 15 bis 20 Minuten auf 93 bis 94°C. Man rührte das Gemisch 10 Minuten bei 93 bis 94°C weiter und destillierte dann unter vermindertem Druck das Trimethylamin, den überschüssigen Isobutyraldehyd sowie 58 Teile Wasser ab. Die verbleibende Lösung von Hydroxypivalinaldehyd wurde dann auf 60°C abgekühlt und unter kräftigem Rühren mit 1,9 Teilen fein pulverisiertem Calciumhydroxid versetzt.

Innerhalb 15 bis 20 Minuten erhitzte sich das Reaktionsgemisch auf Siedetemperatur; die überschüssige Wärme wurde durch Siedekühlung abgeführt. Nach Abklingen der Wärmeentwicklung und kurzem Nachrühren (insgesamt 45 Minuten) wurden bei 70°C 2,5 Teile Ameisensäure (100 %) zugegeben und weitere 10 Minuten gerührt.

Dann wurden 62 Teile Wasser zugesetzt und die Mischung bei 70°C 10 Minuten lang gerührt. Die Phasentrennung erfolgte innerhalb von 15 Minuten, anschließend wurde die untere wäßrige Phase abgelassen und die organische Phase fraktionierend destilliert. Man isolierte 110 Teile 2,2-Dimethyl-1,3-propandiolhydroxypivalinsäure-monoester vom Siedepunkt 120 bis 122°C bei 1,33 mbar, entsprechend 85,9 % der Theorie, bezogen auf den eingesetzten Formaldehyd. Als Destillationsrückstand verblieben 0,7 Teile anorganischer Salze, entsprechend 0,37 %, bezogen auf die abgetrennte organische Phase.

**Beispiel 2**

Ein analog Beispiel 1 hergestelltes rohes Reaktionsgemisch von 2,2-Dimethyl-1,3-propandiolhydroxypivalinsäure-monoester wurde bei 75°C mit 0,6 Teilen Ameisensäure (100 %) versetzt und 10 Minuten lang gerührt. Dann setzte man dem Gemisch 63 Teile Wasser zu, rührte 10 Minuten bei 75°C und trennte nach erfolgter Phasentrennung nach 20 Minuten die untere, wäßrige Phase ab. Die Analyse der organischen Phase ergab einen Gehalt an Salzen von 0,1 % (gerechnet als Calciumformiat). Durch erneute Behandlung der organischen Phase mit 63 Teilen Wasser unter oben genannten Bedingungen reduzierte sich der Salzgehalt der organischen Phase auf 0,02 % (gerechnet als Calcium-formiat). Die organische Phase (157 Teile) hatte einen gaschromatographisch ermittelten Gehalt von 60,2 Gew.-% an 2,2-Dimethyl-1,3-propandiolhydroxypivalinsäure-monoester (entspricht 75 % der Theorie, bezogen auf dem eingesetzten Formaldehyd), die im nächsten Ansatz rückführbare wäßrige Phase der zweiten Extraktion enthielt gaschromatographisch bestimmt (58 Teile) 13,3 Gew.-% 2,2-Dimethyl-1,3-propandiolhydroxypivalinsäure-monoester, entsprechend 6,1 % der Theorie, bezogen auf den eingesetzten Formaldehyd.

**Beispiel 3**

Ein rohes Reaktionsgemisch, das der kalk-katalysierten Tischtschenko-Reaktion von 375 g Hydroxypivalinaldehyd entstammte und daneben noch 12 g Wasser enthielt, wurde bei 70°C mit 10,2 g Eisessig versetzt und 10 min lang gerührt. Anschließend setzte man 120 g Wasser zu, rührte die Mischung bei 70°C 15 min lang und trennte nach erfolgter Phasentrennung die untere, wäßrige Phase ab. Eine analytische Bestimmung des Salzgehalts der organischen Phase ergab einen Wert von 1,42 % (gerechnet als Calciumacetat).

Durch fraktionierende Destillation der organischen Phase isolierte man nach einem Vorlauf von 65 g

unumgesetztem Hydroxypivalinaldehyd 275 g 2,2-Dimethyl-1,3-propandiolhydroxypivalinsäure-monoester vom Siedepunkt 119 bis 121°C bei 1,33 mbar, entsprechend 80 % der Theorie, bezogen auf den umgesetzten Hydroxypivalinaldehyd.

**Beispiel 4**

Ein analog Beispiel 1 hergestelltes rohes Reaktionsgemisch von 2,2-Dimethyl-1,3-propandiolhydroxypivalinsäure-monoester wurde bei 75°C mit 2,5 Teilen Ameisensäure und 63 Teilen Wasser vermischt und 15 min lang gerührt. Nach erfolgter Phasentrennung wurde die untere Phase abgetrennt, die organische Phase bei 75°C 15 min lang erneut mit 63 Teilen Wasser behandelt, worauf der Salzgehalt der organischen Phase dann 0,07 % betrug (gerechnet als Calcium-formiat).

Nach Phasentrennung bei 75°C und destillativer Abtrennung leicht-siedender Komponenten unter vermindertem Druck wurde der rohe 2,2-Dimethyl-1,3-propandiolhydroxypivalinsäure-monoester destillativ bei 1,33 mbar über einen SAMBAY-Verdampfer isoliert.

Der hierbei anfallende Sumpf betrug 1,1 % bezogen auf das Destillat und verursachte keinerlei technische Probleme im Verlauf der Destillation.

**Patentansprüche**

1. Verfahren zur Gewinnung von Hydroxypivalinsäureneopentylglykolester (2,2-Dimethyl-1,3-propandiol-hydroxypivalinsäure-monoester), aus Reaktionsgemischen, die man bei der Umsetzung von Hydroxypivalinaldehyd in Gegenwart von Calcium-, Barium- oder Strontiumhydroxid als Katalysator und anschließende Säurebehandlung erhält, durch Abtrennung der hierbei entstehenden Salze und destillative Aufarbeitung des verbleibenden Gemisches auf den Hydroxypivalinsäureneopentylglykolester, dadurch gekennzeichnet, daß man im Reaktionsgemisch zur Entfernung der Salze einen Wassergehalt von mindestens 20 % und höchstens 60 % einstellt und die Salze bei Temperaturen von 50 bis 100°C extrahiert.

2. Verfahren nach Anspruch 1, dadurch gekennzeichnet, daß man es auf solche Gemische anwendet, die mit Ameisensäure behandelt werden.

3. Verfahren nach Anspruch 2, dadurch gekennzeichnet, daß man 0,4 bis 3,0 mol Ameisensäure pro Mol Katalysator verwendet.

4. Verfahren nach den Ansprüchen 1 und 2, dadurch gekennzeichnet, daß der pH-Wert des Reaktionsgemisches vor der Extraktion auf 3 bis 7 eingestellt wird.

5. Verfahren nach Anspruch 1, dadurch gekennzeichnet, daß man die Extraktion bei Temperaturen von 60 bis 80, insbesondere 65 bis 75°C durchführt.

6. Verfahren nach Anspruch 1 und 2, dadurch gekennzeichnet, daß man im Reaktionsgemisch einen Wassergehalt von mindestens 20 und höchstens 40 % einstellt.

7. Verfahren nach Anspruch 1, dadurch gekennzeichnet, daß man die wäßrige Phase, die nach wiederholter Extraktion anfällt, in die erste Extraktionsstufe eines weiteren Ansatzes einbringt.

**Claims**

1. A process for isolating neopentyl glycol hydroxypivalate (2,2-dimethyl-propane-1,3-diol monohydroxypivalate) from a reaction mixture, obtained by reaction of hydroxypivalaldehyde in the presence of calcium hydroxide, barium hydroxide or strontium hydroxide as the catalyst, and subsequent acid treatment, by removing the salts thereby formed and working up the residual mixture by distillation to give neopentyl glycol hydroxypivalate, wherein, to remove the salts, the water content in the reaction mixture is adjusted to from 20 to 60%, and the salts are extracted at from 50 to 100°C.

2. A process as claimed in claim 1, when applied to a mixture which is treated with formic acid.

3. A process as claimed in claim 2, wherein from 0.4 to 3.0 moles of formic acid are used per mole of catalyst.

4. A process as claimed in claim 1 and 2, wherein the pH of the reaction mixture is brought to 3 to 7 before extraction.

5. A process as claimed in claim 1, wherein the extraction is carried out at from 60 to 80°C, especially from 65 to 75°C.

6. A process as claimed in claims 1 and 2, wherein the water content of the reaction mixture is adjusted to from 20 to 40%.

7. A process as claimed in claim 1, wherein the aqueous phase obtained after repeated extraction is introduced into the first extraction stage of a further batch.

**Revendications**

1. Procédé de récuperation d'ester néopentylglycolique d'acide hydroxypivalique (monoester du 2,2-diméthyl-1,3-propanediol de l'acide hydroxypivalique), à partir de mélanges réactionnels que l'on obtient dans la transformation d'aldéhyde hydroxypivalique en présence d'hydroxyde de strontium, de barium ou de strontium servant de catalyseur, suivie de traitement par un acide, par séparation des sels qui se forment dans ces conditions et traitement par distillation du mélange residuaire pour l'ester néopentylglycolique d'acide hydroxypivalique, caractérisé en ce qu'on règle dans le mélange réactionnel, pour l'élimination des sels, une teneur en eau de 20 % au moins et de 60 % au plus et en ce qu'on extrait les sels à une température de 50 à 100° C.

2. Procédé selon la revendication 1, caractérisé en ce qu'on l'applique à des mélanges qui sont traités par l'acide formique.

3. Procédé selon la revendication 2, caractérisé en ce qu'on utilise 0,4 à 3,0 mol d'acide formique par mol de catalyseur.

4. Procédé selon la revendication 1 ou 2, caractérisé en ce que le pH du mélange réactionnel est réglé à 3-7 avant l'extraction.

5. Procédé selon la revendication 1, caractérisé en ce qu'on effectue l'extraction à une température de 60 à 80° C, en particulier de 65 à 75° C.

6. Procédé selon la revendication 1 ou 2, caractérisé en ce qu'on règle, dans le mélange réactionnel, une teneur en eau de 20 % au moins et de 40 % au plus.

7. Procédé selon la revendication 1, caractérisé en ce qu'on introduit la phase aqueuse, qui est obtenue après extraction repetée, dans la première phase d'extraction d'une nouvelle charge.